# EUROPEAN PATENT APPLICATION

(11) **EP 4 726 731 A1**
(43) Date of publication of application: **15.04.2026**
(21) Application number: 24383114.6
(22) Date of filing: 11.10.2024
(51) Int. Cl.: G16H 20/40, G16H 40/20, A61B 34/10, G06Q 10/06, G06Q 10/1093

(54) **COMPUTER-IMPLEMENTED METHOD FOR OPTIMISING THE RESOURCES ASSOCIATED WITH THE SURGICAL ACTIVITY OF A HOSPITAL**

(71) Applicant: Bidea Avant S.L., 20014 San Sebastian (ES)
(72) Inventor: EGURROLA MARTÍN, Luis Fernando, 20800 ZARAUTZ (ES); LOSANTOS ALBACETE, Mario Jesus, 30008 MURCIA (ES)
(74) Representative: Galbaian S.Coop.

(57) **Abstract**

Computer-implemented method (1) for optimising the resources associated with the surgical activity of a hospital. The method (1) predicts the estimated duration of pending surgical interventions on the surgical waiting list using a model selected from a plurality of duration prediction models configured to calculate the estimated duration of pending interventions from the actual duration of the surgical interventions already performed in historical data. The selected model has the smallest deviation between the actual duration of the surgical interventions already performed in the historical data and the estimated duration calculated for said surgical interventions by means of the corresponding duration prediction model.

## Description

### TECHNICAL FIELD

The present invention relates to computer-implemented methods for optimising the resources associated with the surgical activity of a hospital.

### PRIOR ART

An operating room is a scarce and costly resource that involves a number of specific professional groups, and where specific equipment, which can be very expensive, is used. Furthermore, its use is conditioned by a changing schedule and activity. Optimising theis use is essential to meet the demands of waiting lists, which is a necessity in any hospital and health system.

Currently, the use of various methods to optimise the use of operating rooms in a hospital system is known. However, the methods used for this purpose apply large safety margins, do not estimate unusual cases well, and are not flexible, making it difficult to evaluate several possible scenarios using different options using these methods.

### DISCLOSURE OF THE INVENTION

The object of the invention is to provide a computer-implemented method for optimising the resources associated with the surgical activity of a hospital, as defined in the claims.

The computer-implemented method of the invention is a method for optimising the resources associated with the surgical activity of a hospital. The method comprises a step of configuring a study period, wherein a study period defined by a start date and an end date is configured. Next, the method comprises a step of receiving a surgical waiting list, wherein a surgical waiting list comprising a plurality of pending surgical interventions is received, such that each pending surgical intervention is assigned a type of procedure, a level of urgency, a deadline for said surgical intervention, and a plurality of resources, wherein said resources may have a value assigned for each surgical intervention. Next, the method comprises a step of receiving calendars, wherein a calendar with the public holidays in the study period, and an availability calendar of each resource are received, and a step of receiving historical data of surgical interventions, wherein historical data of surgical interventions already performed is received, comprising for each surgical intervention already performed, the date of intervention, the type of procedure, the actual duration of said surgical intervention, and the actual value of each resource for said surgical intervention. Next, the method comprises a step of configuring a category of surgical interventions, wherein the category refers to the type of procedure, or to the combination of the type of procedure with one or more resources associated with the surgical intervention, and a step of ordering the waiting list, wherein the surgical interventions on the waiting list are ordered from highest to lowest priority according to their level of urgency and their deadline, resulting in an ordered waiting list.

Next, the method of the invention comprises an iterative planning step, comprising a step of estimating the duration and a scheduling step which are carried out for each surgical intervention on the ordered waiting list starting from the highest priority surgical intervention to the lowest priority one. In the step of estimating the duration, an estimated duration for the corresponding surgical intervention is obtained, the step of estimating the duration comprising a step of calculating the duration, and a step of selecting the duration prediction model. In the step of calculating the duration, a plurality of duration prediction models are used, each configured to calculate an estimated duration for the corresponding surgical intervention from the actual duration of the surgical interventions already performed in the historical data with the same category as that of the corresponding surgical intervention, such that each duration prediction model uses a specific algorithm. In the step of selecting the duration prediction model, for each duration prediction model a deviation is calculated between the actual duration of the surgical interventions already performed in the historical data with the same category as that of the corresponding surgical intervention and the estimated duration calculated for said surgical interventions by means of the respective duration prediction model, such that the duration prediction model with the smallest deviation is selected from all models. Thus, the estimated duration calculated by the duration prediction model selected in the step of selecting the duration prediction model is assigned as the estimated duration for the corresponding intervention. In the scheduling step of the method, the corresponding surgical intervention is scheduled as early as possible in the study period, based on the estimated duration calculated for the corresponding surgical intervention, and the availability of the resources assigned to said surgical intervention.

Finally, the method comprises a step of generating a report, wherein a report is generated showing the scheduling of each surgical intervention on the waiting list.

The method of the invention makes it possible to estimate the duration of the pending interventions on the surgical waiting list accurately, in such a way that the scheduling of these interventions optimises the use of the resources necessary to perform them.

These and other advantages and features of the invention will become apparent in view of the figures and the detailed description of the invention.

### DESCRIPTION OF THE DRAWINGS

Figure 1 shows the flow diagram of a preferred embodiment of the method of the invention.
Figure 2 shows the flow diagram of another embodiment of the method of the invention.
Figure 3 shows a schematic depiction of an embodiment of the computer 500 configured to carry out the method of the invention.

### DETAILED DISCLOSURE OF THE INVENTION

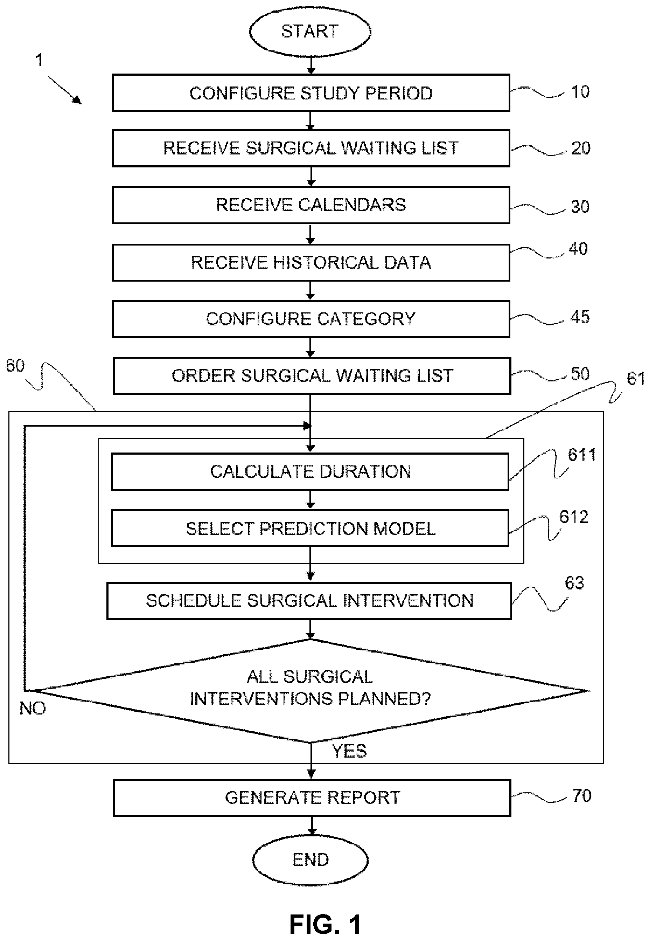

Figure 1 shows the flow diagram of a preferred embodiment of method 1 of the invention.

The computer-implemented method of the invention is a method for optimising the resources associated with the surgical activity of a hospital. The method 1 comprises a step of configuring a study period 10, wherein a study period defined by a start date and an end date is configured. Next, the method 1 comprises a step of receiving a surgical waiting list 20, wherein a surgical waiting list comprising a plurality of pending surgical interventions is received, such that each pending surgical intervention is assigned a type of procedure, a level of urgency, a deadline for said surgical intervention, and a plurality of resources, wherein said resources may have a value assigned for each surgical intervention. The method 1 of the invention comprises a step of receiving calendars 30, wherein a calendar with the public holidays in the study period, and an availability calendar of each resource are received, a step of receiving historical data of surgical interventions 40, wherein historical data of surgical interventions already performed is received comprising for each surgical intervention already performed, the date of intervention, the type of procedure, the actual duration of said surgical intervention, and the actual value of each resource for said surgical intervention, a step of configuring a category of surgical interventions 45, referring by category to the type of procedure, or to the combination of the type of procedure with one or more resources associated with the surgical intervention, and a step of ordering the waiting list 50, wherein the surgical interventions on the waiting list are ordered, from highest to lowest priority, according to their level of urgency and their deadline, resulting in an ordered waiting list.

Next, the method 1 of the invention comprises an iterative planning step 60, comprising a step of estimating the duration 61 and a scheduling step 63 which are carried out for each surgical intervention on the ordered waiting list starting from the highest priority surgical intervention to the lowest priority surgical intervention. In the step of estimating the duration 61 an estimated duration for the corresponding surgical intervention is obtained. Said step of estimating the duration 61 comprises a step of calculating the duration 611 and a step of selecting the duration prediction model 612. In the step of calculating the duration 611 a plurality of duration prediction models are used, each configured to calculate an estimated duration for the corresponding surgical intervention from the actual duration of the surgical interventions already performed in the historical data with the same category as that of the corresponding surgical intervention, such that each duration prediction model uses a specific algorithm. In the step of selecting the duration prediction model 612, for each duration prediction model a deviation is calculated between the actual duration of the surgical interventions already performed in the historical data with the same category as that of the corresponding surgical intervention and the estimated duration calculated for said surgical interventions by means of the respective duration prediction model, such that the duration prediction model with the smallest deviation is selected from all models, and the estimated duration calculated by the selected duration prediction model is assigned as the estimated duration for the corresponding intervention. In the scheduling step 63 of method 1, the corresponding surgical intervention is scheduled as early as possible in the study period, based on the estimated duration calculated for the corresponding surgical intervention, and the availability of the resources assigned to said surgical intervention.

Finally, the method 1 of the invention comprises a step of generating a report 70, wherein a report is generated in which the scheduling of each surgical intervention on the waiting list is shown.

In a hospital there is a volume of people who already have a diagnosis and require surgery. The surgical waiting list includes the surgical interventions already identified in the hospital or health system to be optimised, and which are pending to be performed. Each intervention on the waiting list is assigned a type of procedure that identifies the type of surgery to be performed. The different types of procedure have a worldwide classification standard called "International Statistical Classification of Diseases and Related Health Problems" (ICD), although the method 1 of the invention may use this classification or another, as appropriate for the hospital or health system in question. In the context of the invention, the term intervention refers to a surgical intervention. Similarly, the term waiting list will refer to a surgical waiting list.

In addition, each intervention on the surgical waiting list has a level of urgency associated with it which allows the interventions to be classified according to the urgency with which they should be performed. Additionally, the interventions on the surgical waiting list have a deadline associated with them that identifies the date by which the intervention should be performed. Method 1 of the invention orders the surgical interventions on the surgical waiting list, from highest to lowest priority, according to their level of urgency and their deadline, resulting in an ordered waiting list. In one embodiment of method 1 of the invention, the method 1 orders the interventions by prioritising those whose deadline is closest in time, in such a way that among those with the same deadline, those with the highest level of urgency are prioritised. However, in other embodiments of the invention, the criteria for ordering the surgical waiting list may be different. Finally, each intervention on the waiting list has a plurality of resources associated with it that are necessary for the intervention to be performed. These resources may be, among others, an operating room, a surgeon, a type of recovery, an anaesthesiologist, etc. When a surgical intervention is identified and included in the surgical waiting list, it is common that when entering the information related to that intervention, the value of one or more resources for that intervention is identified. For example, sometimes, for a particular intervention on the waiting list, a specific surgeon is identified to perform that intervention, for example, by his first name and surname, or by his corresponding surgeon identifier. Similarly, for an intervention on the waiting list, the specific operating room may be identified, identified by its corresponding operating room identifier, the recovery area, also identified by its corresponding name or by its corresponding recovery area identifier, etc., although these resources need not have a preassigned value on the waiting list.

In the method 1 of the invention a study period is configured which is a period of time between a start date and an end date. In addition, the method 1 of the invention receives a calendar with the public holidays in the study period. Thus, the method 1 of the invention schedules the interventions of the waiting list by optimising the resources necessary to perform them on the non-holiday days of the study period. Additionally, the method 1 of the invention receives an availability calendar of each resource, namely a calendar of each resource value, identifying the days on which a resource is available and its available working hours. For example, method 1 will have the calendar associated with each surgeon, such that method 1 will schedule the interventions to be performed by that surgeon on the days and working hours of that surgeon.

The historical data of surgical interventions already performed comprise for each surgical intervention already performed the data showing how it actually took place, i.e. the date on which the intervention took place, the type of procedure that was performed, its actual duration, and the actual value of each resource in said surgical intervention, data which are accessible to the method 1 of the invention.

Starting from the interventions on the ordered waiting list, the method 1 tries to plan each of these interventions by processing them one at a time, starting with the surgical intervention with the highest priority and ending with the one with the lowest priority. In other words, the method 1 iterates, processing one intervention from the waiting list in each iteration, such that in the iterative planning step 60, the corresponding intervention is the intervention being processed in the current iteration. Thus, the first thing that the method 1 carries out for the corresponding intervention, or in other words, for the intervention being processed, is an estimation of the duration of said intervention based on the historical data of the surgical interventions already performed. In order to carry out the calculation of the estimated duration, the method 1 is based on the actual duration of the interventions already performed in the historical data whose category is equal to the category of the intervention being processed. The method 1 of the invention allows configuring the category to be used in the method 1. The use of the category is particularly advantageous since it makes it possible to group interventions that resemble each other into groups, and to estimate the duration of a pending intervention on the waiting list from the actual durations of the interventions already performed in the historical data belonging to the same group. Thus, in one embodiment of the method 1 of the invention where the category is configured to be the type of procedure, in order to calculate the estimated duration of the corresponding intervention the method 1 will take into account the actual duration of all those interventions in the historical data whose type of procedure value is equal to the type of procedure value of the corresponding intervention. In another embodiment where the category is configured to be the combination of the type of procedure and the surgeon, to calculate the duration of the corresponding intervention the method 1 will take into account the actual duration of all those interventions in the historical data whose type of procedure value is equal to the type of procedure value of the corresponding intervention and whose value for the surgeon resource is equal to the value of the surgeon resource associated with the corresponding intervention. In other embodiments, the category may take other values and may be configured as the combination of the type of procedure and the operating room, or others. The use as a category of the combination of the type of procedure and the operating room resource is particularly advantageous, given that it allows for a more accurate estimation of the duration of those surgical interventions on the waiting list that, despite having the same type of procedure, have to be performed with specific equipment that is only available in one or more operating rooms. By means of the method 1 of the invention, it is possible to generate different schedules of the interventions on the surgical waiting list depending on how the category is configured, which makes it possible to preview and evaluate in advance the different scenarios that would occur depending on the configuration made in each case for the category. In the context of the invention, the expression "the surgical interventions already performed in the historical data with the same category as that of the corresponding intervention on the waiting list" refers to the surgical interventions already performed in the historical data whose actual value for the type of procedure is the same as the value assigned to the type of procedure of the intervention on the waiting list, so that if the category has been configured as a combination of the procedure type and a resource, this expression refers to the surgical interventions already performed in the historical data whose actual value for the procedure type is the same as the value assigned to the procedure type of the intervention on the waiting list, and whose actual value for the resource is the same as the value assigned to the resource in the intervention on the waiting list.

In the step of calculating the duration 611, the method 1 uses a plurality of duration prediction models, such that by means of each of them it calculates a different estimated duration, given that each of said prediction models uses a specific algorithm to carry out said calculation. However, although each of said prediction models uses a different algorithm to carry out the calculation of the estimated duration, all of them do so on the basis of the actual duration of the interventions already performed in the historical data whose category is equal to the category of the intervention being processed.

In the step of selecting the duration prediction model model 612, the method 1 calculates for each duration prediction model a deviation between the actual duration of the surgical interventions already performed in the historical data with the same category as that of the corresponding surgical intervention and the estimated duration calculated for said surgical interventions by the respective duration prediction model, the duration prediction model with the smallest deviation being selected from all models, and the estimated duration calculated by the duration model selected in the step of selecting the duration prediction model 612 being assigned as the estimated duration for the corresponding intervention. Various methods for calculating such a deviation are now known. The method 1 of the invention is not intended to be limited to the use of a particular method, and any method known in the prior art may be used for the calculation of such a deviation.

In the method of the invention, for each pending intervention on the waiting list, several prediction models are used to calculate its estimated duration, selecting from among all of them the one with the smallest deviation so that it is precisely this prediction model that is used to estimate the duration of said intervention. As a result, for each pending intervention on the waiting list, the method dynamically selects the prediction model that provides the most accurate estimated duration for that intervention. Thus, for each of the pending interventions on the waiting list, depending on the value for the category in that intervention (e.g. the value of the procedure type for that intervention in the case where the category has been configured as the procedure type, or the value of the procedure type and the value of the operating room for that intervention in the case where the category has been configured as the combination of the procedure type and the operating room resource), the prediction model that provides the most accurate duration estimate for that intervention is selected. Therefore, the method 1 of the invention makes it possible to estimate the duration of the pending interventions on the surgical waiting list accurately, in such a way that the scheduling of said interventions optimises the use of the resources necessary to perform them.

In the scheduling step 63, the method 1 schedules the corresponding surgical intervention as early as possible in the study period, based on the estimated duration calculated for the corresponding surgical intervention at the step of estimating the duration 61, and the availability of the resources assigned to said surgical intervention.

Finally, in the step of generating a report 70, the method 1 generates a report showing the scheduling of each surgical intervention on the waiting list. This schedule shows for each intervention on the waiting list whether it could be scheduled in the study period, given that it may happen that certain interventions cannot be scheduled due to the unavailability of the resources necessary to perform them. In one embodiment of the method 1, for those that could be scheduled, the report will show at least the estimated duration for each one, the day and time it was scheduled, the type of procedure, and the value assigned to the resources associated with it.

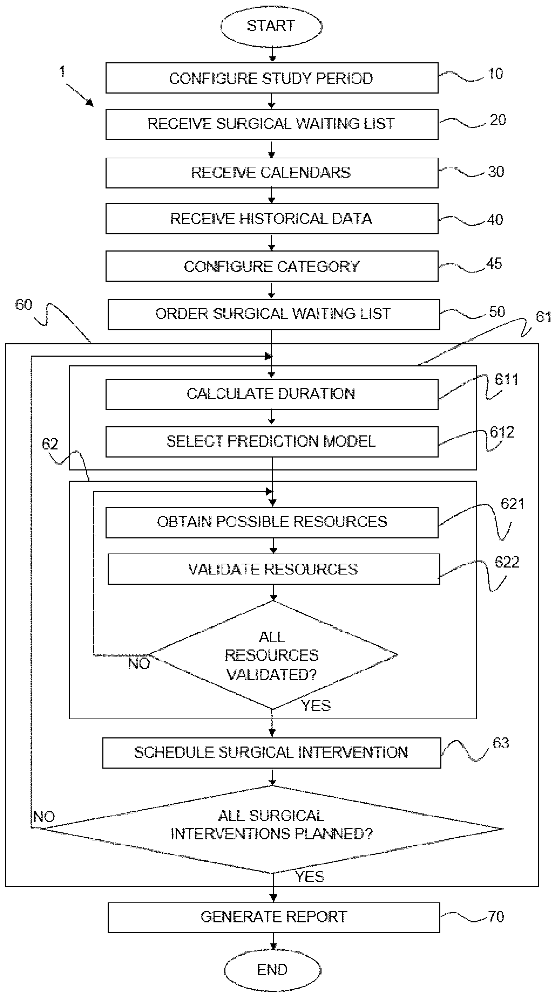

Figure 2 shows the flow diagram of another embodiment of the method 1 of the invention, wherein the iterative planning step 60 carried out for each surgical intervention on the ordered waiting list comprises an iterative step of validating resources 62 after the step of estimating the duration 61, comprising for each resource associated with the corresponding surgical intervention a step of obtaining possible resources 621, and a step of validating resources 622.

In the step of obtaining possible resources 621 of the method 1, the number of surgical interventions in the historical data with the same category as the corresponding surgical intervention that have been performed for each value of the corresponding resource is calculated, with possible resource values being those for which the number of surgical interventions is greater than zero when the number of surgical interventions in the historical data with the same category as that of the corresponding surgical intervention is greater than a predefined support value.

In the step of validating resources 622 of the method 1, for those surgical interventions on the surgical waiting list for which a value is assigned to the corresponding resource, said value is considered to be a valid value, if this value is one of the possible resource values obtained in the step of obtaining possible resources 621 for the corresponding surgical intervention and the corresponding resource, and for those surgical interventions on the waiting list whose corresponding resource is not assigned a value, the possible resource value obtained in the step of obtaining possible resources 621 for which the number of surgical interventions in the historical data with the same category as that of the corresponding surgical intervention is maximum is assigned as the valid value for said resource.

The pending interventions on the waiting list may have a value assigned to one or more of their associated resources. The method 1 of the invention allows validating whether the values assigned to said resources are valid or not, based on the surgical interventions already performed in the historical data. For this purpose, in the step of obtaining possible resources 621 the method 1 calculates whether there is a sufficient number of interventions in the historical data with the same category as that of the corresponding intervention, i.e. the intervention being processed. To do this, it compares said number of interventions with a support value, a value from which it is considered that the number of interventions in the historical data is sufficient to validate the values assigned to the resources associated with the corresponding intervention. If the number calculated in the step of obtaining possible resources 621 is sufficient and the corresponding resource of the corresponding intervention has an assigned value, the method 1 checks if said value is one of the possible resource values obtained in the step of obtaining possible resources 621. If the assigned value is among the possible resource values, the method 1 considers said value as a valid value, thus validating the assignment of said value for the corresponding resource of the corresponding intervention. However, if the value assigned to the corresponding resource of the corresponding intervention of the waiting list is not among the possible resource values obtained in the step of obtaining possible resources 621, the method 1 would not validate it as a valid value for said resource. Thus, the method 1 warns of the assignment of a value to a resource of an intervention on the waiting list for which there are not enough interventions in the historical data to support it as a valid value for said resource, for the category value used. That is, for such a category value, it would be one or more other values of the corresponding resource that have been assigned to the resource in the surgical interventions already performed in the historical data. Conversely, if the corresponding resource of the corresponding intervention on the waiting list does not have an assigned value, the method 1 assigns to it that possible resource value obtained in the step of validating resources 622 for which there is a greater number of interventions in the historical data that support it as the most probable value for that resource, for the value of the category used. In this way, all the interventions on the waiting list have a value assigned to each of their resources, which makes it possible to schedule them in the scheduling step 63, taking into account both the estimated duration calculated for said intervention, as well as the availability of the associated resources, i.e. the resources identified with the assigned resource values.

In one embodiment of the method 1 of the invention, the support value is defined per resource, or per resource value. That is, a value may be defined for each resource that is validated in the iterative step of validating resources 62, for example, a support value to validate the values assigned to the surgeon resource of pending interventions and a different support value for the operating room resource, or a support value may be defined for each resource value that is validated, such that the support value to validate a first value assigned to the surgeon resource of an intervention may be different from the support value used to validate a second value assigned to the surgeon resource of another intervention.

In one embodiment of the method 1 of the invention, the resources are the operating room, the surgeon, and the recovery area. The recovery area identifies the destination to which the patient to whom the intervention is to be performed will be transferred after the intervention has been performed. The method 1 of the invention allows the availability of such a resource to be taken into account when scheduling. However, in other embodiments of the method 1 of the invention, other resources can be assigned to the surgical interventions on the waiting list, and therefore also to the surgical interventions already performed in the historical data.

In one embodiment of method 1 of the invention, in the step of estimating the duration 61 the duration of the exchange time and the duration of the recovery time for each intervention on the surgical waiting list are also estimated. The exchange time is the time required to prepare an operating room after an intervention has been completed so that the operating room is ready for another intervention to be performed therein. The recovery time associated with each intervention is the time the patient undergoing the intervention is required to stay in the assigned recovery area. The historical data of surgical interventions comprises for each surgical intervention already performed the actual duration of the exchange time that was required after each intervention to prepare the operating room for the intervention that was performed next, and the actual duration of the recovery time that the patient undergoing each intervention spent in the recovery area.

In said embodiment, in the step of estimating the duration 61 the estimated duration of the exchange time for the corresponding surgical intervention is also obtained. Said step comprises a step of calculating the duration 611, wherein a plurality of duration prediction models are used, each configured to calculate an estimated duration of the exchange time for the corresponding surgical intervention from the actual duration of the exchange time of surgical interventions already performed in the historical data with the same category as that of the corresponding surgical intervention, such that each duration prediction model uses a specific algorithm, and a step of selecting the duration prediction model 612, wherein for each duration prediction model a deviation is calculated between the actual duration of the exchange time of the surgical interventions already performed in the historical data with the same category as that of the corresponding surgical intervention and the estimated duration of the exchange time calculated for said surgical interventions by means of the respective duration prediction model, selecting from all models the duration prediction model with the smallest deviation, assigning as estimated duration of the exchange time for the corresponding intervention the estimated duration of the exchange time calculated by the selected duration prediction model.

Likewise, the step of estimating the duration 61 also provides the estimated duration of the recovery time for the corresponding surgical intervention. Said step comprises a step of calculating the duration 611, wherein a plurality of duration prediction models are used, each configured to calculate an estimated duration of the recovery time for the corresponding surgical intervention from the actual duration of the recovery time of surgical interventions already performed in the historical data with the same category as that of the corresponding surgical intervention, such that each duration prediction model uses a specific algorithm, and a step of selecting the duration prediction model 612, wherein for each duration prediction model a deviation is calculated between the actual duration of the recovery time of the surgical interventions already performed in the historical data with the same category as that of the corresponding surgical intervention and the estimated duration of the recovery time calculated for said surgical interventions by means of the respective duration prediction model, the duration prediction model with the smallest deviation being selected from all models, and the estimated recovery time duration calculated by the selected duration prediction model being assigned as the estimated duration of the recovery time for the corresponding intervention.

In the scheduling step 63, the scheduling of the interventions on the waiting list will take into account both the estimated duration of the corresponding intervention and the duration of the exchange time. The scheduling will also take into account the estimated duration of the recovery time when analysing the availability of the recovery area.

In one embodiment of the method 1 of the invention, in the step of selecting the duration prediction model 612, the mean squared error is used to calculate the deviation.

In one embodiment of method 1 of the invention, the duration prediction models are based on the calculation of the mean, the median, the mode, the simple moving average (SMA), and/or on the use of a neural network previously trained with the historical data of the surgical interventions already performed. Thus, one of the duration prediction models used in the step of calculating the duration 611 calculates the estimated duration of the corresponding surgical intervention as the mean of the actual durations of the surgical interventions already performed in the historical data with the same category as that of the corresponding intervention. However, another duration prediction model used in said step of the method 1 calculates said estimated duration as the median of said actual durations, another model does so by calculating the mode of said actual durations, and another one by calculating the simple moving average (SMA) of said actual durations. However, the above duration prediction models are not intended to be a limitation of the method 1 of the invention, and other models based on other statistical algorithms known in the prior art may be used in the step of calculating the duration 611 of the method 1. Another duration prediction model used in the step of calculating the duration 611 is a neural network previously trained with the historical data of the surgical interventions already performed so that, from the data corresponding to a pending intervention on the surgical waiting list, it is capable of providing an estimated duration for the same. However, in the step of calculating the duration 611 of method 1, other models based on machine learning known in the prior art could be used.

In one embodiment of the method 1 of the invention, each surgical intervention on the waiting list has a service associated with it, the method 1 comprising a step of configuring assignment templates before the iterative planning step 60, wherein a plurality of assignment templates are received, each comprising an assignment of the services to the operating rooms by shifts and/or days for a specific period of time, the scheduling step 63 being configured so that the assignment of surgical interventions on the surgical waiting list to the operating rooms takes into account the assignment of services of at least one assignment template. In hospitals, it is common for a given operating room to be assigned during a morning, afternoon, and/or night shift to a service or a unit in the hospital. Some of the possible hospital services are listed below: paediatric cardiology, cardiac surgery, paediatric cardiac surgery, paediatric digestive surgery, general colorectal surgery, general and digestive surgery, and general endocrine surgery. For example, a specific assignment template comprises the following assignments for a given operating room: general and digestive surgery on Monday morning and afternoon shifts, general endocrine surgery on Tuesday morning shifts, general colorectal surgery on Wednesday and Thursday morning and afternoon shifts, and paediatric cardiology on Friday mornings. The use of assignment templates facilitates the scheduling of the interventions on the waiting list, and contributes to a sensible scheduling, in which the activity of an operating room during a shift or several consecutive shifts is performed by the same service of the hospital, and therefore by the same team of professionals. This contributes to a better organisation of the activity of said professionals, optimising their available time.

In one embodiment, the method 1 of the invention comprises a step of configuring session templates before the iterative planning step 60, wherein a plurality of session templates are received, each session template comprising an assignment of a plurality of surgical interventions with the same type of procedure per shift and/or day for each operating room and subset of surgeons, the scheduling step 63 being configured so that the assignment of surgical interventions on the surgical waiting list to the operating rooms takes into account the assignment of surgical interventions of at least one session template. In hospitals, it is common for a given operating room to be used during a morning, afternoon, and/or night shift to perform interventions with the same type of procedure. As an example, a specific session template comprises an assignment of five consecutive interventions with a procedure type, or main procedure of value 39.90 corresponding to the insertion of a renal stent during the morning shift of a given operating room. The use of session templates facilitates the scheduling of the interventions on the waiting list and helps to optimise the duration of the interventions, as they are all of the same type of procedure, and are usually performed by the same surgeon, who is an expert in that procedure.

In one embodiment, the method 1 of the invention comprises a step of predicting a waiting list of expected surgical interventions, wherein by using a prediction model of expected surgical interventions, a plurality of expected surgical interventions are estimated, which although unknown at the time of performing the method 1, are expected to be scheduled in the study period based on the surgical interventions in the historical data, and wherein said expected surgical interventions are incorporated into the surgical waiting list. The prediction model of expected surgical interventions is a machine learning based model previously trained with the historical data of the surgical interventions already performed to provide a prediction of the expected surgical interventions during the study period. The estimated surgical interventions are incorporated into the surgical waiting list, so that in the iterative planning step 60 of the method 1, they are scheduled in the study period.

In one embodiment, the method 1 of the invention comprises a step of predicting a waiting list of urgent surgical interventions, wherein by using a prediction model of urgent surgical interventions, a plurality of urgent surgical interventions that are expected to be scheduled in the study period are estimated based on the surgical interventions in the historical data, and wherein said urgent surgical interventions are incorporated into the surgical waiting list. The prediction model of urgent surgical interventions is a machine learning based model previously trained with historical data of the surgical interventions already performed to provide a prediction of urgent surgical interventions during the study period. The urgent surgical interventions are incorporated into the surgical waiting list, so that in the iterative planning step 60 of method 1, they are scheduled in the study period.

In one embodiment, the method 1 of the invention comprises a step of receiving a list of possible referrals, wherein a list of possible referrals comprising the type of procedure and the hospital to which said surgical intervention may be referred to is received, such that in the scheduling step 63 the scheduling of the surgical interventions on the surgical waiting list is performed taking into account said list of possible referrals.

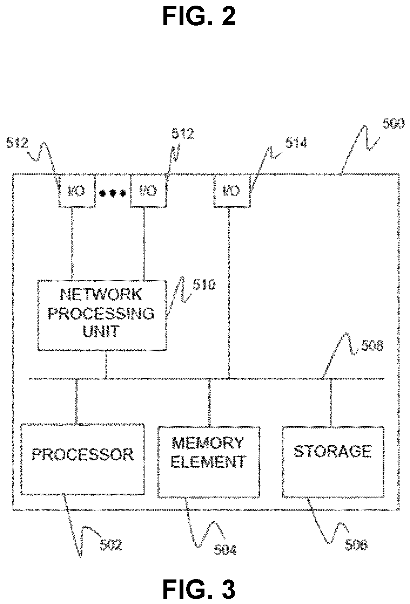

Figure 3 shows a schematic depiction of an embodiment of a computer 500 configured to implement the method 1 of the invention. In at least one embodiment, the computer 500 used to implement the method 1 of the invention may include one or more processors 502, one or more memory elements 504, storage 506, a bus 508, one or more network processor units 510 interconnected with one or more network input/output (I/O) interfaces 512, one or more I/O interfaces 514, and a computer program.

The computer program comprises a plurality of instructions which, when executed by the processor 502, cause the processor 502 to execute the steps of the method 1 of the invention. In at least one embodiment, the processor or processors 502 are at least a hardware processor configured to execute various tasks, operations, and/or functions for the computer 500 according to the software and/or the instructions configured for the computer 500, for example, in the computer program.

In at least one embodiment, the memory element(s) 504 and/or storage 506 are configured to store data, information, software, and/or instructions associated with the computer 500, and/or logic configured for the memory element(s) 504 and/or storage 506. In one embodiment of the computer 500, the computer program is stored in any combination of memory element(s) 504 and/or storage 506.

In one embodiment of method 1 of the invention, data necessary to implement the method 1, such as the surgical waiting list, schedules, and the historical data of surgical interventions, is stored in any combination of memory element(s) 504 and/or storage 506.

In one embodiment of method 1 of the invention, the report generated with the scheduling of the surgical interventions on the waiting list is stored in a memory element 504 and/or storage 506.

In at least one embodiment, the bus 508 may be configured as an interface that allows one or more elements of the computer 500 to communicate with each other to exchange information and/or data. The bus 508 may be implemented with any architecture designed to exchange control, data, and/or information between processors, memory/storage elements, peripheral devices, and/or any other hardware and/or software components that may be configured for the computer 500. In at least one embodiment, the bus 508 may be implemented as a rapid interconnection housed in the core, potentially using shared memory between processes (e.g., logic), which may allow efficient communication routes between processes.

In various embodiments, the network processor unit(s) 510 may enable communication between the computer 500 and other systems, entities, etc., via the network I/O interface(s) 512 (wired and/or wireless). In various embodiments, the network processor unit(s) 510 may be configured as a combination of hardware and/or software, such as one or more Ethernet controllers and/or interface controllers or cards, Fibre Channel (e.g., optical) driver(s) and/or controller(s), wireless receivers/transmitters/transceivers, baseband processor(s)/modem(s) and/or other similar network interface driver(s) and/or controller(s) now known or hereinafter developed to enable communications between the computer 500 and other systems, entities, etc. to facilitate the operations for the various embodiments of method 1 described herein. In various embodiments, the network I/O interface(s) 512 may be configured as one or more Ethernet ports, Fibre Channel ports, any other I/O port(s) and/or antenna(s)/antenna array(s) now known or hereafter developed. Thus, network processor unit(s) 510 and/or network I/O interface(s) 512 may include interfaces suitable for receiving, transmitting and/or otherwise communicating data and/or information in a network environment.

The I/O interfaces 514 allow input and output of data and/or information with other entities that may be connected to the computer 500. For example, the I/O interfaces 514 may provide a connection to external devices such as a keyboard, keypad, touch screen, and/or any other suitable input and/or output device now known or developed in the future. In some cases, the external devices may also include computer-readable (non-transitory) storage media such as database systems, USB memories, portable optical or magnetic disks, and memory cards. Still in some cases, external devices may be a mechanism for displaying data to a user, such as, for example, a computer monitor, display screen, or the like.

In various embodiments, the computer program can include instructions that, when executed, cause the processor(s) 502 to perform operations, which can include, but are not limited to, providing general computer control operations, interacting with other entities, systems, etc. described herein, maintain and/or interact with stored data, information, parameters, etc. (e.g., memory element(s), storage, data structures, databases, tables, etc.); combinations thereof; and/or the like to enable execution of operations necessary for the implementation of the method 1 of the invention.

In some cases, the computer program of the present embodiments may be available via a non-transitory computer-readable storage medium (e.g., magnetic or optical media, magnetooptical media, CD-ROM, DVD, memory devices, etc.). In some cases, non-transitory computer-readable storage media may also be removable. Other examples may include optical and magnetic disks, USB memories and smart cards that can be inserted into and/or otherwise connected to a computer for transfer to another computer-readable storage medium.

In one embodiment, the computer program runs in a distributed environment, i.e., part of the computer program runs on a first computer 500, and part of the computer program runs on a second computer, both computers 500 communicating with each other.

A second aspect of the invention relates to a computer program comprising a plurality of instructions which, when the program is executed by the computer 500, cause the computer 500 to carry out the steps of the method 1 of the invention.

A third aspect of the invention relates to a computer-readable storage medium comprising a plurality of instructions which, when executed by the computer 500, cause the computer 500 to carry out the steps of the method 1 of the invention.

## Claims

1. Computer-implemented method for optimising the resources associated with the surgical activity of a hospital, comprising
- a step of configuring a study period (10), wherein a study period defined by a start date and an end date is configured,
- a step of receiving a surgical waiting list (20), wherein a surgical waiting list comprising a plurality of pending surgical interventions is received, such that each pending surgical intervention is assigned a type of procedure, a level of urgency, a deadline for said surgical intervention, and a plurality of resources, wherein said resources may have a value assigned for each surgical intervention,
- a step of receiving calendars (30), wherein a calendar with the public holidays in the study period, and an availability calendar of each resource are received,
- a step of receiving historical data of surgical interventions (40), wherein historical data of surgical interventions already performed is received, comprising for each surgical intervention already performed, the date of the intervention, the type of procedure, the actual duration of said surgical intervention, and the actual value of each resource for said surgical intervention,
- a step of configuring a category of surgical interventions (45), wherein the category refers to the type of procedure, or to the combination of the type of procedure with one or more resources associated with the surgical intervention,
- a step of ordering the waiting list (50), wherein the surgical interventions on the waiting list are ordered from highest to lowest priority according to their level of urgency and their deadline, resulting in an ordered waiting list,
- an iterative planning step (60), comprising the following steps which are carried out for each surgical intervention on the ordered waiting list starting from the highest priority surgical intervention to the lowest priority surgical intervention,
- a step of estimating the duration (61), wherein an estimated duration for the corresponding surgical intervention is obtained, comprising
- a step of calculating the duration (611), wherein a plurality of duration prediction models is used, each configured to calculate an estimated duration for the corresponding surgical intervention from the actual duration of the surgical interventions already performed in the historical data with the same category as that of the corresponding surgical intervention, such that each duration prediction model uses a specific algorithm, and
- a step of selecting the duration prediction model (612), wherein for each duration prediction model a deviation is calculated between the actual duration of the surgical interventions already performed in the historical data with the same category as that of the corresponding surgical intervention and the estimated duration calculated for said surgical interventions by means of the respective duration prediction model, such that the duration prediction model with the smallest deviation is selected from all models, the estimated duration calculated by the selected duration prediction model being assigned as the estimated duration for the corresponding intervention, and
- a scheduling step (63), wherein the corresponding surgical intervention is scheduled as early as possible in the study period, based on the estimated duration calculated for the corresponding surgical intervention, and the availability of the resources assigned to said surgical intervention, and
- a step of generating a report (70), wherein a report is generated in which the scheduling of each surgical intervention on the waiting list is shown.

2. Computer-implemented method according to claim 1, wherein the iterative planning step (60) carried out for each surgical intervention on the ordered waiting list comprises an iterative step of validating resources (62) after the step of estimating the duration (61), comprising for each resource associated with the corresponding surgical intervention
- a step of obtaining possible resources (621), wherein the number of surgical interventions in the historical data with the same category as the corresponding surgical intervention that have been performed for each value of the corresponding resource is calculated, wherein possible resource values are those for which said number of surgical interventions is greater than zero when the number of surgical interventions in the historical data with the same category as that of the corresponding surgical intervention is greater than a predefined support value; and
- a step of validating resources (622), wherein
- for those surgical interventions on the waiting list whose corresponding resource is assigned a value, said value is considered a valid value if said value is one of the possible resource values obtained in the step of obtaining possible resources (621) for the corresponding surgical intervention and the corresponding resource, and
- for those surgical interventions on the waiting list whose corresponding resource is not assigned a value, the possible resource value obtained in the step of obtaining possible resources (621) for which the number of surgical interventions in the historical data with the same category as that of the corresponding surgical intervention is the maximum is assigned as the valid value for said resource.

3. Computer-implemented method according to claim 3, wherein the support value is defined per resource, or per resource value.

4. Computer-implemented method according to any of the preceding claims, wherein the resources are the operating room, the surgeon, and the recovery area.

5. Computer-implemented method according to any of the preceding claims, wherein in the step of estimating the duration (61) the exchange time and the recovery time for each intervention on the surgical waiting list are also estimated.

6. Computer-implemented method according to any of the preceding claims, wherein in the step of selecting the duration prediction model (612) the mean squared error is used to calculate the deviation.

7. Computer-implemented method according to any of the preceding claims, wherein the duration prediction models are based on the calculation of the mean, median, mode, or simple moving average (SMA), and/or on the use of a neural network previously trained with the historical data of surgical interventions already performed.

8. Computer-implemented method according to any of the preceding claims, wherein each surgical intervention on the waiting list has a service associated with it, the method comprising a step of configuring assignment templates before the iterative planning step (60), wherein a plurality of assignment templates are received, each assignment template comprising an assignment of the services to the operating rooms by shifts and/or days for a specific period of time, the scheduling step (63) being configured so that the assignment of surgical interventions on the surgical waiting list to the operating rooms takes into account the assignment of services of at least one assignment template.

9. Computer-implemented method according to any of the preceding claims, comprising a step of configuring session templates before the iterative planning step (60), wherein a plurality of session templates are received, each session template comprising an assignment of a plurality of surgical interventions with the same type of procedure by shift and/or day for each operating room and subset of surgeons, the scheduling step (63) being configured so that the assignment of surgical interventions on the surgical waiting list to the operating rooms takes into account the assignment of surgical interventions of at least one session template.

10. Computer-implemented method according to any of the preceding claims, comprising a step of predicting a waiting list of expected surgical interventions, wherein by using a prediction model of expected surgical interventions a plurality of expected surgical interventions are estimated, which although unknown at the time of executing the method (1), are expected to have to be scheduled in the study period based on the surgical interventions in the historical data, and wherein said expected surgical interventions are incorporated into the surgical waiting list.

11. Computer-implemented method according to any of the preceding claims, comprising a step of predicting a waiting list of urgent surgical interventions, wherein by using a prediction model of urgent surgical interventions a plurality of urgent surgical interventions that are expected to have to be scheduled in the study period are estimated based on the surgical interventions in the historical data, and wherein said urgent surgical interventions are incorporated into the surgical waiting list.

12. Computer-implemented method according to any of the preceding claims, comprising a step of receiving a list of possible referrals, wherein a list of possible referrals comprising the type of procedure and the hospital to which said surgical intervention can be referred to is received, such that in the scheduling step (63) the scheduling of surgical interventions from the surgical waiting list is performed taking into account said list of possible referrals.

13. Computer program comprising a plurality of instructions which, when the program is executed by a computer (500), cause the computer (500) to carry out the steps of the method (1) according to any of the preceding claims.

14. Non-transitory computer-readable storage medium comprising a plurality of instructions which, when executed by a computer (500), cause the computer (500) to carry out the method steps (1) according to any of claims 1 to 12.
